# EUROPEAN PATENT APPLICATION

(11) **EP 4 186 488 A1**
(43) Date of publication of application: **31.05.2023**
(21) Application number: 21210636.3
(22) Date of filing: 26.11.2021
(51) Int. Cl.: A61K 8/365, A61K 8/49, A61K 8/9789, A61Q 15/00, A61Q 19/02, A61K 8/67

(54) **A TOPICAL COMPOSITION**

(71) Applicant: Unilever IP Holdings B.V., 3013 AL Rotterdam (NL)
(72) Inventor: The designation of the inventor has not yet been filed
(74) Representative: Warner, Guy Jonathan

(57) **Abstract**

The present invention relates to a personal care composition which gives even tone and brightness to skin. It more particularly relates to a topical composition which is effective against hyperpigmentation especially in the under-arm region. This is achieved through applying a composition on to skin comprising an extract of *Salvia officinalis* and a second active selected from one or both of hydroxystearic acid and a vitamin B3 compound and a topically acceptable carrier.

## Description

### Field of the Invention

The present invention relates to a personal care composition which gives even tone and brightness to skin. It more particularly relates to a topical composition which is effective against hyperpigmentation especially in the under-arm region.

### Background of the Invention

Most people consider skin appearance as one of the key indicators of their own beauty and health. Having an even skin tone which is bright and free of blemishes is thus desired by many. The degree and evenness of pigmentation of the skin is affected by factors like age, hormonal changes, occurrence of acne and exposure to sunlight and pollution. These can cause spots or freckles, and hyper-pigmentation on certain localised areas of skin. The axilla or the under-arm, although not always visible on the outside also undergoes higher level of darkening as compared to the neighbouring region. Many consumers wish to have an even and bright skin tone in the underarm region.

The present inventors have been working in this area for a long time and have also filed several patent applications and produced various cosmetic products which are in the market. In the present invention, they were specifically looking for an effective solution to the problem of uneven skin colour especially in the under-arm region. They are aware that the skin in the underarm region is sensitive in many respects including application of chemicals which may be harsh on some people. The problem therefore was to find a solution to providing even skin colour with use of mild actives which do not irritate the skin but at the same time deliver the desired even tone and glow to the skin.

They are aware that extracts from plants like those of *Salvia officinalis* are known for lightening skin. The present applicant has published patents on effective delivery of extract of *Salvia officinalis* which is also known as sage. EP2706975B1 discloses a skin lightening composition comprising a) 0.1 to 5 % by weight disodium fumarate; b) 0.01 to 5 % by weight an extract of a plant material comprising 50 to 100 % ursolic acid; and c) a cosmetically acceptable vehicle. Although this patent is effective in delivering sage extract efficiently, there is still a need in the art to deliver the benefits of this extract using lower amounts. The present inventors took the approach of trying to achieve this by including materials commonly used in the art. This approach not only ensures lower cost of the product but also enables lower processing and stability issues due to use of lower concentration of the actives. The present inventors through their understanding of the various mechanisms to deliver melanin inhibition and through extensive experimentation have hit upon two actives that interact synergistically to ensure better efficacy of even skin tone, which is especially of use in underarm skin colour. The two actives viz. (i) a vitamin B3 compound e.g. niacinamide and (ii) hydroxystearic acid have been known for use in skin products and so can be safely incorporated in such products without too much extra effort in understanding their stability and possible detrimental interaction with other commonly used skin care actives. To the knowledge of the present inventors, a combination of the claimed actives herein has not been used or known to deliver the benefit of the present invention.

It is thus an object of the present invention to provide for a solution to the problem of pigmentation and uneven skin appearance.

It is another object of the present invention to provide for such a solution specifically for the under-arm region.

It is yet another object of the present invention to provide a solution which is cost effective.

### Summary of the Invention

The first aspect of the present invention relates to a topical composition comprising (a) an extract of *Salvia officinalis;* (b) a second active selected from one or both of hydroxystearic acid and a vitamin B3 compound; and (c) a topically acceptable carrier.

Another aspect of the present invention relates to a method of providing even and glowing tone to skin comprising the step of applying a composition of the first aspect on to the desired skin.

### Detailed Description of the Invention

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

The topical composition of the invention is meant to be used for personal care or for cosmetic use and could also be referred to as a personal care composition or a cosmetic composition. By a "personal care composition" as used herein, is meant to include a composition for topical application i.e external surfaces of the skin and/or hair of humans. Such a composition may be classified as leave-on or rinse off, and includes any product applied to a human body for improving appearance, cleansing, odour control or general aesthetics. The composition is preferably of the leave-on type. The composition of the present invention can be in the form of a liquid, lotion, cream, foam, stick, roll-on, gel or through an aerosol containing composition. Preferred compositions include lotion, cream, stick or as a roll-on form or through an aerosol containing composition.

"Skin" as used herein is meant to include skin on the face and body (e.g., neck, chest, back, arms, underarms, hands, legs and scalp) and especially to the underarm region.

The composition of the present invention includes an extract of *Salvia officinalis* which is also known as sage. The plant material for providing the extract used in this invention is *Salvia officinalis* with CAS No 84082-79-1**.**

*Salvia officinalis* is often referred to by the common name of Garden sage or Common sage. It is a small perennial evergreen subshrub, with woody stems, grayish leaves, and blue to purplish flowers. It is a member of the family Lamiaceae and is native to the Mediterranean region. It is cultivated in many European countries and in North America. It has been used in medicinal preparations and in foods. In the present invention, the leaves and soft stems of *Salvia officinalis* are used for extracting the desired actives, preferably the leaves. Sage leaf contains the following chemical class of compounds: catechin-type tannins, phenolic acids, flavonoids, essential oil and triterpenoids which includes oleanolic and ursolic acids.

A preferred process of preparing the extract of the plant material for use in the composition of the invention is prepared using a process comprising the steps of (a) contacting the plant material *Saliva officinalis* with a non-polar solvent selected from hexane, ethyl acetate, toluene, chloroform, diethyl ether or methylene chloride to prepare a mixture; and (b) separating the solvent from the mixture to prepare the extract. The separation of the solvent may be carried out by any known method. A preferred method is evaporation of the solvent under vacuum, preferably followed by filtration, washing and drying to a powder.

The extract may be further purified by a step of washing the extract with a solvent which may be hexane, acetone or a mixture thereof. Further purification steps may involve dissolving the extract in a basic solution e.g. alkaline ammonia followed by filtering the solution free of insoluble material and neutralizing the solution with acid to a neutral pH to precipitate the desired extract which may be further washed with distilled water and dried to a powder which contains ursolic acid.

The plant extract preferably comprises 50 to 100% ursolic acid, more preferably 60 to 95%, further preferably 60 to 85% ursolic acid. A preferred aspect provides oleonolic acid to be present in 15 to 40% by weight of the plant extract. In a highly preferred aspect, both ursolic acid and oleonolic acid are present. By the term extract is meant a product in dry form which could be in powder, granule or in the form of lumps. The percentage of fatty acid in the extract of plant material throughout this specification, unless mentioned otherwise, is on dry weight basis.

The sage extract is preferably included in the composition of the invention in 0.01 to 5% more preferably 0.1 to 1.5% by weight of the composition.

The composition of the invention comprises a second active selected from one or both of hydroxystearic acid and a vitamin B3 compound.

It is preferred that the hydroxystearic acid is 10-hydroxystearic acid, 12-hydroxystearic acid or trihydoxystearic acid (e.g. 9,10,13-trihydroxystearic acid) or trihydroxy stearin or compounds that yield one or more molecules of hydroxystearic acid or hydroxystearate on their breakdown like mono, di or tri ester of glycerol with hydroxystearic acid. Of these, 10-hydroxystearic acid, 12-hydroxystearic acid and 9,10,13-trihydroxystearic acid are more preferred, 12-hydroxystearic acid (12-HSA) being most preferred. 12-HSA has the structure as given below: 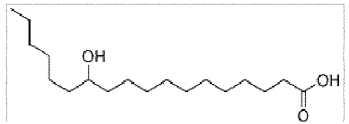 Hydroxystearic acid is preferably included in 0.01 to 5%, more preferably 0.05 to 3%, further more preferably 0.1 to 2% by weight of the composition.

The second active may preferably be a Vitamin B3 compound. The vitamin B3 compound is selected from one or more of niacin, nicotinic acid, isonicotinamide, picolinamide, and nicotinamide (which is also known as niacinamide). It is preferably niacinamide. Niacinamide also known as pyridine-3-carboxamide is the active, water soluble form of vitamin B3. When included the composition of the present invention comprises an effective amount of niacinamide, typically in a concentration of 0.001 to 10%, preferably at least 0.01%, more preferably at least 0.1%, still more preferably at least 1% by weight of the composition; Niacinamide is preferably not more than 9%, more preferably not more than 8%, still more preferably not more than 7%, yet more preferably not more than 6%, or even not more than 5% by weight of the composition.

It is preferred that the weight ratio of hydroxystearic acid to sage extract is in the range of 5:1 to 0.5:1. It is preferred that the weight ratio of vitamin B3 compound to sage extract is in the range of 1000:1 to 5:1.

Without wishing to be bound by theory the inventors believe that the combination of inhibitory effect of sage extract on the ET1 signaling mechanisms required for melanin production and inhibition of melanosome transfer by HSA/ Vitamin B3 compound bring about superior skin brightening effect.

When the composition of the invention is delivered as a sunscreen composition that also delivers skin even tone it may comprise an organic sunscreen selected from one or both of a UVA sunscreen and a UVB sunscreen. Sunscreens include those materials commonly employed to block ultraviolet light. Illustrative compounds are the derivatives of PABA, cinnamate and salicylate. For example, avobenzophenone (Parsol 1789^{®}) octyl methoxycinnamate and 2-hydroxy-4-methoxy benzophenone (also known as oxybenzone) can be used. Octyl methoxycinnamate and 2-hydroxy-4-methoxy benzophenone are commercially available under the trademarks, Parsol MCX and Benzophenone-3, respectively. The exact amount of sunscreen employed in the compositions can vary depending upon the degree of protection desired from the sun's UV radiation. Additives that reflect or scatter the sun rays may also be employed. These additives include oxides like zinc oxide and titanium dioxide.

The composition of the present invention may further comprise a cosmetically acceptable vehicle, which may act as diluents, dispersants. and/or carriers for the actives used in the composition, so as to facilitate their distribution when the composition is applied to the skin. The cosmetically acceptable vehicle suitable for use in the present invention may be aqueous, anhydrous or an emulsion; aqueous or an emulsion, especially water-in-oil or oil-in-water emulsion being most preferred. Water when present typically makes up the balance of the composition. Preferably water is present in a concentration of 5 to 99%, more preferably from 20 to 80%, still more preferably from 40 and 80% by weight of the composition.

The composition of the present invention may be delivered in a cream, lotion or gel form preferably in cream form. A preferred format for the solid form of the composition is a cream, further more preferably one which has a vanishing cream base. Vanishing cream base is one which comprises 3 to 25 wt% fatty acid. Optionally, the composition may comprise 0.1 to 10 wt% soap. When included, the fatty acid is preferably a C10 to C22 fatty acid, more preferably a C16 to C18 fatty acid. Most preferably the fatty acids are stearic acid or palmitic acid or a mixture thereof and the soap is preferably the potassium salt of the fatty acid mixture. The fatty acid is often hystric acid which is substantially (generally about 90 to 95 %) a mixture of 45 % stearic acid and 55 % palmitic acid. The most preferred cream is one having 3 to 25 wt% fatty acid and 0.1 to 10 wt% soap.

Preferably, the composition comprises emollients. Examples of emollients that may be used in the leave-on composition include stearyl alcohol, glyceryl monoricinoleate, mink oil, isopropyl isostearate, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils such as dimethylpolysiloxane, din-butyl sebacate, isopropyl myristate, isopropyl palmitate, isopropyl stearate, butyl stearate, polyethylene glycol, triethylene glycol, lanolin, cocoa butter, corn oil, cotton seed oil, olive oil, palm kernel oil, rape seed oil, safflower seed oil, evening primrose oil, soybean oil, sunflower seed oil, avocado oil, sesame seed oil, coconut oil, arachis oil, castor oil, acetylated lanolin alcohols, petroleum jelly, mineral oil, butyl myristate, isopropyl linoleate, lauryl lactate, myristyl lactate, decyl oleate, myristyl myristate and mixtures thereof.

Preferably, the composition comprises solvents. Examples of solvents that may be used in the composition include ethyl alcohol, isopropanol, acetone, ethylene glycol mono ethyl ether, diethylene glycol mono butyl ether, diethylene glycol mono ethyl ether and mixtures thereof. The composition may comprise polyhydric alcohols which may be selected from one or more of glycerine, 1,3-butylene glycol, propylene glycol, 1,3-propanediol, pentylene glycol, hexylene glycol, and sorbitol.

Preferably, the composition comprises powders. Examples of powders that may be used in the composition include chalk, talc, fullers earth, kaolin, starch, gums, colloidal silica sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate and mixtures thereof.

Preferably, the composition comprises preservatives to protect against the growth of potentially harmful microorganisms. Examples of ingredients that may be used as preservatives in the composition include alkyl esters of para-hydroxybenzoic acid, hydantoin derivatives, propionate salts, and a variety of quaternary ammonium compounds. More preferably, ingredients that may be used as preservative in the composition are sodium benzoate, iodopropynyl butyl carbamate, methylisothiazolinone, iodopropynylbutylcarbamate, phenoxyethanol, methyl paraben, propyl paraben, imidazolidinyl urea, sodium dehydroacetate, ethylhexylglycerin, benzyl alcohol, alkane diols and mixtures thereof. When present in the composition, preservatives are added preferably in an amount 0.001 to 5 wt%, more preferably 0.01 to 3 wt% and most preferably 0.02 to 2 wt%, even most preferably 0.25 to 1.5%.

Preferably, the composition comprises a range of other optional ingredients that include antioxidants, binders, buffering agents, colorants, astringents, fragrance, opacifying agents, conditioners, exfoliating agents, pH adjusters, skin sensates, skin soothing agents, and skin healing agents.

When the composition of the invention is delivered as product of use in the underarm it may be delivered using a topically acceptable carrier that is usually used to deliver deodourant or antiperspirant products.

An especially preferred form of a product which is delivered as a deodourant or an antiperspirant format involves a cosmetically acceptable base which is anhydrous. Such anhydrous compositions are useful for deodorant products especially for application in the underarm region. The composition comprises a topically acceptable base which is preferably anhydrous. By an anhydrous base is meant that water content in the composition is less than 5wt%, preferably less than 2 wt%, more preferably less than 1 wt% and optimally absent from the composition. To enable this, the anhydrous base preferably comprises a silicone compound, a polyhydric alcohol or a wax.

The pH of the composition is preferably higher than 3.5 more preferably in the range of 4 to 7. The pH of the composition of the invention is measured using the following procedure:
Equal volumes of the composition and model ionic sweat (pH 6.1) are mixed, and the pH value is measured using an accurate range pH test paper.

The anhydrous composition of the invention preferably comprises a polyhydric alcohol. Polyhydric alcohol is also referred to in short as polyol. A polyhydric alcohol as per the present invention is a compound having two or more hydroxyl groups. Suitable class of polyhydric alcohols that may be included in the composition of the invention are monomeric polyols, polyalkylene glycols or sugars. Preferred monomeric polyols are glycol; alkylene glycol e.g. propylene glycol; glycerol; or xylitol, more preferably propylene glycol.

Suitable polyalkylene glycols are polyethylene glycol or polypropylene glycol. Sugars for inclusion in the invention could be monomeric, dimeric, trimeric or of the polymeric form. Preferred sugars include glucose, fructose, mannose, sucrose, threitol, erythritol, sorbitol, mannitol, galactitol, adonitol, dextran, or cyclodextrin. Of these the more preferred sugars are glucose, fructose, sucrose, sorbitol, mannitol, adonitol, dextran, or cyclodextrin.

Other components commonly included in conventional compositions may also be incorporated in the composition of the present invention. Such components include skin care agents such as emollients, humectants and skin barrier promoters; skin appearance modifiers such as skin lightening agents and skin smoothing agents; anti-microbial agents, in particular organic anti-microbial agents, and preservatives.

The composition of the invention can be applied cosmetically and topically to the skin, broadly speaking, by one of two methods. Some consumers prefer one method and some others, the other method. In one method, sometimes called a contact method, a composition is wiped across the surface of the skin, depositing a fraction of the composition as it passes. In the second method, sometimes called the non-contact method, the composition is sprayed from a dispenser held proximate to the skin, often in an area of about 10 to 20 cm². The spray can be developed by mechanical means of generating pressure on the contents of the dispenser, such as a pump or a squeezable sidewall or by internally generated pressure arising from a fraction of a liquefied propellant volatilising, the dispenser commonly being called an aerosol.

There are broadly speaking two classes of contact compositions, one of which is liquid and usually applied using a roll-on dispenser or possibly absorbed into or onto a wipe, and in the second of which the desired actives are distributed within a carrier liquid that forms a continuous phase that has been gelled. In one variation, the carrier fluid comprises a solvent for the actives and in a second variation, the actives remain as a particulate solid that is suspended in an oil, usually a blend of oils.

Many different materials have been proposed as gellant for a continuous oil phase, including waxes, small molecule gelling agents and polymers. They each have their advantages and of them, one of the most popular class of gellant comprises waxes, partly at least due to their ready availability and ease of processing, including in particular linear fatty alcohol wax gellants. A gelled composition is applied topically to skin by wiping it across and in contact with the skin, thereby depositing on the skin a thin film.

The nature of the film depends to a significant extent on the gellant that is employed. Although wax fatty alcohols have been employed as gellant for many years, and are effective for the purpose of gelling, the resultant product is rather ineffective at improving the visual appearance of skin, and in particular underarm skin, to which the composition has been applied. This problem has been solved by including ameliorating materials for example, di or polyhydric humectants and/or a triglyceride oil.

Liquid compositions that are applicable from a roll-on broadly speaking can be divided into two classes, namely those in which the actives are suspended in a hydrophobic carrier, such as a volatile silicone and those in which the actives are dissolved in a carrier liquid. The latter has proven to be more popular. There are mainly two sorts of dissolving carrier liquid, namely carriers that are predominantly alcoholic, which is to say the greater part of the dissolving carrier fluid comprises ethanol and the second class in which the carrier liquid is mainly water. The former was very popular because ethanol is a mild bactericide in its own right, but its popularity waned because it stings, especially if the surface onto which the composition has been applied has been damaged or cut, such as can easily arise during shaving or other de-hairing operations.

The second class of formulations that is an alternative to alcoholic formulations comprise a dispersion of water-insoluble or very poorly water soluble ingredients in an aqueous solution of the actives. Herein, such compositions will be called emulsions. Roll-on emulsions commonly comprise one or more emulsifiers to maintain a distribution of the water-soluble ingredients.

The composition of the invention may be delivered through an aerosol composition which comprises a propellant in addition to the other ingredients described hereinabove. Commonly, the propellant is employed in a weight ratio to the base formulation of from 95:5 to 5:95. Depending on the propellant, in such aerosol compositions the ratio of propellant to base formulation is normally at least 20:80, generally at least 30:70, particularly at least 40:60, and in many formulations, the weight ratio is from 90:10 to 50:50. A ratio range of from 70:30 to 90:10 is sometimes preferred.

Propellants herein generally are one of three classes; i) low boiling point gasses liquifided by compression, ii) volatile ethers and iii) compressed non-oxidising gases.

Class i) is conveniently a low boiling point material, typically boiling below -5°C, and often below -15°C, and in particular, alkanes and/or halogenated hydrocarbons. This class of propellant is usually liquefied at the pressure in the aerosol canister and evaporates to generate the pressure to expel the composition out of the canister. Examples of suitable alkanes include particularly propane, butane or isobutane. The second class of propellant comprises a very volatile ether of which the most widely employed ether hitherto is dimethyl ether. This propellant can advantageously be employed at relatively low weight ratio of propellant to base formulation, for example to as low as 5:95. It can also be employed in admixture with, for example, compressible/liquefiable alkane gasses. The third class of propellant comprises compressed non-oxidising gasses, and in particular carbon dioxide or nitrogen. Inert gases like neon are a theoretical alternative.

When the composition of the invention is delivered in a roll-on, a firm solid or a stick format, the topically acceptable base comprises a hydrophobic carrier or an aqueous carrier. The hydrophobic carrier in such cases may comprise a silicone compound, or a wax. When the composition comprises a propellant it is delivered as an aerosol.

The composition of the invention preferably additionally comprises a fragrance. By a fragrance is meant a molecule or a composition comprising a group of molecules that produces a pleasant odour. The composition preferably comprises a fragrance in 0.1 to 3% by weight of the composition.

The packaging for the composition of this invention can be a patch, bottle, tube, roll-ball applicator, propellant driven aerosol device, squeeze container or lidded jar.

In another aspect, the invention relates to a method of providing even and glowing tone to skin comprising the step of applying a composition of the invention on to the desired skin. A preferred skin surface in this method is the axilla. The method is preferably non-therapeutic.

In yet another aspect, the invention relates to use of a combination of extract of salvia officinalis and a second active selected from one or both of hydroxystearic acid and a vitamin B3 compound for providing synergistic reduction in melanin content in skin.

Extract of *Salvia officinalis* for use in the present invention was purchased from the USA as supplied by Sabinsa Corporation, USA. The supplier sourced the sage grown in Morocco and Russia.

The invention will now be illustrated by means of the following non-limiting examples.

### Examples

In-vitro studies on the effect of the combination of the actives on melanin inhibition using melanin content assay (MCA) was used. The method is described below.

### MCA Method

Human primary melanocytes were grown in melanocytes growth medium (MGM) with human melanocyte growth supplement. 500 µL/well (5X10⁴ cells/well) of this solution was plated in a 24 wells plate and incubated in an incubator (Thermo Scientific, Model 3111) at 37°C with 5% CO₂ atmosphere. After 24 hours of incubation, cell cultures were treated with the lead actives either dissolved in water or DMSO. The cells were again incubated for 72 hours in an incubator (Thermo Scientific, Model 3111; conditions: 5% CO2, at 37°C).

Comparative vehicle controls of 0.1 % (v/v) DMSO or media control (for aqueous actives) were also set up simultaneously. At the end of the incubation period, cell viability was determined using the calcein method.

### Calcein method

Briefly, cell culture spent media was removed and cells washed once with 0.4 ml of 1x PBS-Ca-Mg solution. Fresh 1 mM calcein-AM was added (0.4 ml/well), including to control wells without cells. Plates were covered with aluminium foil and incubated for 30 min. at 37 °C in the regular CO₂ incubator. Calcein fluorescence was then measured (excitation at 490 nm and emission at 520 nm) in TECAN M1000 plate reader.

After calcein fluorescence readings were obtained, drained the cells and added fresh 0.15 ml of 1N NaOH (in 10% DMSO) per well. Cells were lysed by resuspension and incubation (60 °C/1 hr.). Then, 0.12 ml of this lysate was transferred to a fresh 396-well plate and measured OD₄₀₅ₙₘ in a TECAN M1000 plate reader (estimate of relative melanin content).

### Calculation for melanin content:

The spectrophotometric OD at 405nm for untreated cells (No active) is considered as 100% melanin content, with respect to this, treatment (active) percentage is calculated.

The data on the % inhibition of melanin content is shown in Table -1 below.

**Table -1 :**

| Example | Active(s), wt% | % melanin inhibition | Std. Dev |
|---|---|---|---|
| A | Sage extract 0.0001% | 23.7 | 2.2 |
| B | Niacinamide 0.00122% | 7.4 | 4.4 |
| C | Niacinamide 0.0122% | 9.2 | 4.1 |
| D | Niacinamide 0.061% | 6.1 | 3.6 |
| 1 | Sage extract 0.0001% + | 34.7 | 2.2 |
| 2 | Sage extract 0.0001% Niacinamide 0.061% | 46.2 | 0.8 |
| 3 | Sage extract 0.0001% + Niacinamide 0.061% | 45.4 | 0.6 |
| E | 12-HSA 0.00015% | 4.1 | 5.2 |
| 4 | Sage extract 0.0001% + 12-HSA 0.00015% | 32.2 | 1.4 |

The data in the table above indicates that there is synergistic interaction between sage extract and 12-HSA and between sage extract and niacinamide to give enhanced melanin inhibition. This is indicative of providing enhanced even skin tone with improved brightness.

It is to be understood that the experiments described above were conducted in an invitro assay to evaluate the synergistic melanin inhibition behaviour. It is expected that the concentrations to be actually used to prepare a composition for topical use would be vastly different. The concentrations could be orders of magnitude higher due to the following reasons that affect the difference in concentration in the bulk as compared to that at the cellular level. The composition may be formulated as an emulsion or a gel with very many additional ingredients. The concentration of the desired actives in the oil phase and in the water phase, is expected to be very different. They may also have very different physical and hydrodynamic properties like partition coefficients, diffusional rates, convective transport rates, rheological properties etc. Therefore, it is expected that the concentrations to be used when formulated as a composition would be very different and possibly much higher than that used in the cellular level experiments.

## Claims

1. A topical composition comprising
(a) An extract of *Salvia officinalis*;
(b) a second active selected from one or both of hydroxystearic acid and a vitamin B3 compound; and
(c) a topically acceptable carrier.

2. A composition as claimed in claim 1 wherein the weight ratio of hydroxystearic acid to extract of *Salvia officinalis* is in the range of 5:1 to 0.5:1.

3. A composition as claimed in any one of the preceding claims wherein the hydroxystearic acid is 12-hydroxystearic acid.

4. A composition as claimed in any one of the preceding claims wherein the weight ratio of vitamin B3 compound to extract of *Salvia officinalis* is in the range of 1000:1 to 5:1.

5. A composition as claimed in any one of the preceding claims wherein the vitamin B3 compound is niacinamide.

6. A method of providing even and glowing tone to skin comprising the step of applying a composition as claimed in any one of the preceding claims on to the desired skin.

7. A method as claimed in claim 6 wherein the desired skin is the axilla.

8. Use of a combination of extract of *Salvia officinalis* and a second active selected from one or both of hydroxystearic acid and a vitamin B3 compound for providing synergistic reduction in melanin content in skin.
